Europäisches Patentamt

European Patent Office

Office européen des brevets

(19)

(11) Publication number: **0 438 146 A2**

## EUROPEAN PATENT APPLICATION

(12)

(21) Application number: **91100508.0**

(22) Date of filing: **17.01.91**

(51) Int. Cl.5: **A61K 37/02**

The title of the invention has been amended (Guidelines for Examination in the EPO, A-III, 7.3).

(30) Priority: **19.01.90 IT 1911090**

(43) Date of publication of application:
**24.07.91 Bulletin 91/30**

(84) Designated Contracting States:
**AT BE CH DE DK ES FR GB GR IT LI LU NL SE**

(71) Applicant: **SCLAVO S.p.A.**
**Via Fiorentina 1**
**I-53100 Siena(IT)**

(72) Inventor: **Parente, Luca**
**Via Fracassi, 20**
**I-53100 Siena(IT)**
Inventor: **Mugridge, Kenneth Gordon**
**Via delle Chiuse, 5**
**I-53010 Casciano di Murlo, Siena(IT)**
Inventor: **Wallace, John**
**RR No. 1**
**Cochrane, AB(CA)**
Inventor: **Keenan, Catherine**
**312 Blackthorn Rd. NW**
**Calgary, AB(CA)**

(74) Representative: **Gervasi, Gemma et al**
**NOTARBARTOLO & GERVASI Srl Viale**
**Bianca Maria 33**
**I-20122 Milan(IT)**

(54) Interleukin-1-beta for treating ulcers.

(57) The use of human interleukin-1$\beta$ as gastroduodenal mucosa protector for treating gastroduodenal, benign, recurring and post-operational ulcers is described, together with pharmaceutical compositions containing a therapeutically effective quantity of human interleukin-1$\beta$.

EP 0 438 146 A2

## NEW PHARMACEUTICAL COMPOSITION FOR PROTECTING THE GASTRODUODENAL MUCOSA

This invention relates to the use of human interleukin-1$\beta$ as gastroduodenal mucosa protector for treating gastroduodenal, benign, recurring and post-operational ulcers, and pharmaceutical compositions containing a therapeutically effective quantity of human interleukin-1$\beta$.

Human interleukin-1$\beta$, known hereinafter as huIL-1$\beta$, is a protein pertaining to the lymphomonokine family which is secreted by activated macrophages. This protein is able to mediate various biological and immune activities (Mizel. S.D., Immunol. Rev., 63, 51-72, 1982; Durum, S.K. et al., Annu. Rev. Immunol., 3, 263-270, 1894). In this respect, huIL-1$\beta$ induces release of interleukin-2 to indirectly stimulate T-cell maturation and hence the production of antibodies, so contributing to amplification of the immune response. In addition, huIL-1$\beta$ stimulates maturation and proliferation of B-cells and, in the case of tissue damage, concurs in the activation of reparation mechanisms by indirectly promoting the growth of fibroblasts. Human IL-1$\beta$ is able to stimulate the production of prostaglandins in various cell types and in particular in the isolated stomach of rats (Mugridge et al., 1989, J. Pharmacol. Exp. Ther., 250, 1162-1188).

It has now been surprisingly found that human interleukin-1$\beta$ when administered to animals is able to exercise a protective activity on the gastroduodenal mucosa without inducing undesirable side effects.

Thus the present invention provides a compound having the amino acid sequence corresponding to that of human interleukin-1$\beta$ for use as active principle for gastroduodenal mucosa protection in pharmaceutical compositions.

The present invention also provides a pharmaceutical composition comprising as active principle a therapeutically effective quantity of human interleukin-1$\beta$ and a pharmacologically acceptable non-toxic sterile carrier.

The present invention further provides a method for treating ulcers in man comprising administering a pharmaceutical composition containing a therapeutically effective quantity of human interleukin-1$\beta$.

Further aspects of the present invention will be apparent from the following description.

The present invention therefore enables pharmaceutical compositions to be formulated for therapeutic use at effective doses for treating ulcers in man, such as gastroduodenal, benign, recurring or post-operational ulcers, or for treating hypertrophic or duodenal chronic gastritis when associated with acid hypersecretion.

The active principle for use in the pharmaceutical composition of the present invention can be chosen from recombinant human interleukin-1$\beta$ and interleukin-1$\beta$ isolated from human leukocytes. Human IL-1$\beta$ can be used as active principle for protection of the gastroduodenal mucosa in formulating pharmaceutical compositions for the preparation of dosage forms suitable for oral, intravenous or parenteral administration.

The quantity of huIL-1$\beta$ contained in a pharmaceutical composition can vary in accordance with various parameters such as the nature of the composition, including the particular compound contained in it, and the method of administration.

However, the concentration of said compound is generally between 0.1 and 10 $\mu$g per kg of body weight per day.

The composition of the present invention can be formulated in unit dosage forms depending on the method of administration. In addition to huIL-1$\beta$ as active principle, the formulations according to the present invention can contain pharmacologically acceptable stabilizers, adjuvants, preservatives, antioxidants, emulsifiers, antiflocculating agents, buffers and disintegrating agents chosen from those compatible with the compounds themselves.

In the case of oral formulations, the pharmaceutical compositions can also contain flavourings as known in pharmaceutics, all formulated in gastro-resistant dosage forms, and typically gastro-resistant capsules, to prevent denaturation and enzymatic degradation of the active principle within the stomach, or in the form of sublingual or perbuccal tablets.

In particular, in the case of oral administration, the human interleukin-1$\beta$ can be formulated in combination with therapeutically effective quantities of a non-steroid anti-inflammatory agent to antagonize the gastrolesive effect of the human interleukin-1$\beta$.

Generally, anti-inflammatory agents are chosen from acetylsalicylic acid (at a dose of 0.5-1.0 g per person per day), indomethacin (at a dose of 100-200 mg per person per day), ibuprofen (at a dose of 0.4-1.2 g per person per day) and piroxicam (at a dose of 10-20 mg per day).

In the case of formulations for parenteral administration, the huIL-1$\beta$ can be administered either in the form of an intravenous injection repeated several times per day or by intermittent intravenous infusion.

For administration by intravenous injection, lyophilized huIL-1$\beta$ can be diluted with physiological solution or other intravenously compatible solution to a suitable final volume and then injected within a time period of not less than 5 minutes.

The purpose of the following examples is to better illustrate the present invention, and must not be interpreted as as limitation on their scope.

EXAMPLE 1

<u>Use of recombinant human interleukin-1$\beta$ as protective agent in gastric ulcer caused by ethanol</u>

Groups of 4-8 non-consanguineous male Wistar rats having a body weight equal or approximately equal to 175-225 g and fasting for 24 hours were treated by intraperitoneal (i.p.) administration with an apyrogenic sterile physiological solution either alone (controls) or containing IL-1$\beta$ concentrations of between 0.01 and 5 $\mu$g/kg of body weight. Two hours after inoculation the animals received by oral administration (intergastric tube) 1 ml of absolute ethanol to induce gastric damage.

Fifteen minutes after the ethanol administration the rats were killed and the gastric damage evaluated in a double blind test by an observer ignorant of the treatment, as described by Wallace and Whittle (1985), Eur. J. Pharmacol., 115, 45-52.

The results were expressed as damaged area as a percentage of the total area.

In the control animals (n = 28) this percentage was 21.0 ± 2.5%. As shown in Table I, treatment with huIL-1$\beta$ reduced the damage in a statistically significant dose-dependent manner.

## TABLE I

| Treatment | dose ($\mu$g/kg) | % reduction in gastric damage |
|---|---|---|
| IL-1 beta | 0.1 | 49.4 ± 5.5* |
| " " | 0.5 | 58.2 ± 1.4* |
| " " | 1.0 | 60.5 ± 3.3* |
| " " | 3.0 | 67.0 ± 1.5* |
| " " | 5.0 | 77.0 ± 2.4** |

The results are expressed as mean value ± standard error.

Significance is calculated with respect to the control group.

* = p < 0.05; ** = p < 0.01.

EXAMPLE 2

Use of recombinant human interleukin-1$\beta$ as protective agent in gastric ulcer caused by indomethacin

Groups of 5 non-consanguineous male Wistar rats having a body weight of approximately 175-225 g and fasting for 24 hours were inoculated by intraperitoneal administration with a dose of 20 mg/kg of indomethacin. The rats were treated intraperitoneally 1 hour before and 1 hour after indomethacin administration with apyrogenic sterile physiological solution either alone or containing human IL-1$\beta$ concentrations of between 0.1 and 3 $\mu$g/kg of body weight.

Three hours after indomethacin administration the rats were killed and the stomach withdrawn and examined as described in Example 1.

3

The results given in Table II are expressed as damaged area as a percentage of the total area, and show that human IL-1$\beta$ reduces damage caused by indomethacin in a dose-dependent manner.

## TABLE II

| Treatment | dose (µg/kg) | damaged area % |
|---|---|---|
| Control | - | 17.5 ± 4.9 |
| IL-1 beta | 0.1 | 13.7 ± 5.0 |
| " " | 1.0 | 8.0 ± 3.0 |
| " " | 3.0 | 7.8 ± 2.0 |

The results are expressed as mean value ± standard error.

EXAMPLE 3

Use of recombinant human interleukin-1$\beta$ as protective agent in duodenal ulcer caused by cysteamine

Two groups of 9 non-consanguineous male Wistar rats having a body weight of approximately 175-225 g and fasting for 24 hours were treated orally at time 0 and after 4 hours with 280 mg/kg of cysteamine. One of the two groups had been treated intraperitoneally with only apyrogenic sterile physiological solution whereas the other group had been treated intraperitoneally with a sterile physiological solution containing 1 µg/kg of IL-1$\beta$.

The animals were killed 24 hours after the initial cysteamine administration and the duodenum was removed and examined by a Leitz model Wild MB stereomicroscope. The lesions were classified from 0 to 5 as follows:

0 = undamaged mucosa; 1 = superficial hyperemia 2 = superficial single ulceration ; 3 = deep single ulceration; 4 = deep multiple ulcerations; 5 = perforated ulcer.

The results shown in Table III show that the IL-1$\beta$ had reduced both the incidence of ulcers and the gravity rating of the lesions.

## TABLE III

| Group | Incidence of ulcers | Lesion rating |
|---|---|---|
| Cysteamine | 9/9 (100%) | 3.6 ± 0.3 |
| Cysteamine + IL-1 β | 3/9 (33%) | 1.8 ± 0.4* |

The results are expressed as mean value ± standard error.

* $p < 0.05$.

**Claims**

1. A compound definable by the amino acid sequence corresponding to that of human interleukin-1β, for use as active principle for gastroduodenal mucosa protection in the preparation of pharmaceutical compositions.

2. A pharmaceutical composition comprising as active principle a therapeutically effective quantity of human interleukin-1β in accordance with claim 1.

3. A pharmaceutical composition as claimed in claim 2, wherein the human interleukin-1β is recombinant IL-1β.

4. A pharmaceutical composition as claimed in claims 2 and 3, comprising a quantity of human interleukin-1β therapeutically effective in mammals including man, excipients and additives suitable for the various formulations.

5. A dosage form for the oral administration of a pharmaceutical composition comprising human interleukin-1β as active principle in accordance with claim 1, wherein said active principle is contained in a quantity of between 0.1 and 10 μg per kg of body weight.

6. A dosage form for the oral administration as claimed in claim 5, containing a therapeutically effective quantity of an antiinflammatory agent chosen from acetylsalicylic acid, indomethacin, ibuprofen and piroxicam.

7. A dosage form for the parenteral administration of a pharmaceutical composition comprising as active principle human interleukin-1β in a quantity of between 0.1 and 10 μg per kg of body weight.